# NEW EUROPEAN PATENT SPECIFICATION

(11) **EP 0 942 708 B2**
(45) Date of publication and mention of the opposition decision: **30.05.2007**
(45) Mention of the grant of the patent: 22.01.2003
(21) Application number: 97950746.4
(22) Date of filing: 26.11.1997
(51) Int. Cl.: A61K 8/04, A61K 8/81, A61Q 1/10

(54) **MASCARA COMPOSITIONS HAVING IMPROVED WEAR AND BEAUTY BENEFITS**
MASKARAZUSAMMENSETZUNGEN MIT VERBESSERTEN VERSCHLIESS - UND SCHÖNHEITSEIGENSCHAFTEN
COMPOSITIONS DE FARD A CILS AVEC BENEFICES AMELIORES POUR LE PORT ET LA BEAUTE

(30) Priority: 27.11.1996 US 757538
(43) Date of publication of application: 22.09.1999
(73) Proprietor: THE PROCTER & GAMBLE COMPANY, Cincinnati, Ohio 45202 (US)
(72) Inventor: BARTHOLOMEY, Edward, Martin, Baltimore, MD 21236 (US); TARANTINO, David, Edmund, Sparks, MD 21152 (US); WALLING, David, William, Parkton, MD 21120 (US)
(74) Representative: Wilding, Richard Alan
(86) International application number: PCT/US1997/021890
(87) International publication number: WO 1998/023251

(56) References cited:
- EP-A- 0 600 445
- WO-A-98/18431
- DE-A- 1 949 740
- GB-A- 2 027 341
- PATENT ABSTRACTS OF JAPAN vol. 12, no. 421 (C-541) [3268] , 8 November 1988 & JP 63 151351 A (SHISEIDO), 23 June 1988,
- P.A.CIULLO: "Magnesium Aluminium Silicate In Water-In-Oil Emulsions" DRUG & COSMETIC INDUSTRY, vol. 126, no. 5, 1980, pages 50-153, XP002063212
- A Formulary of Cosmetic Preparations,Volume One:Decorative cosmetics;1991; Pages 129,131,132 and 133.
- Lexikon der Hilfstoffe für Pharmazie,Kosmetik und angrenzende Gebiete; Third Edition; 1998; Page 1258.
- Vanderbilt Report-Veegum in Color Cosmetics.

## Description

### TECHNICAL FIELD

The present invention relates to emulsion mascara compositions comprising inorganic colloidal materials incorporated into the emulsion's internal phase. These compositions exhibits good film strength while avoiding undesired rheology modification by the addition of inorganic colloidal material. These compositions also exhibit very good resistance to smudging, smearing and flaking once the composition has been applied to the eyelashes.

### BACKGROUND OF THE DISCLOSURE

Eye make-up compositions, including mascara, are significant products in the cosmetics market. Mascara enhances the beauty of the wearer by coating the eye lashes, or in some instances eyebrows, with color. In spite of their beauty enhancing characteristics, conventional eye make-up preparations have been criticized for their failure to maintain the desired affect during long periods of wear. Problems such as staining and smearing (commonly referred to as smudging) and flaking of the mascara from the eyelashes are common complaints.

Thickeners, such as organically treated argyles or clays, are known for use in mascara compositions in order to create desired viscosity of the composition, modify its flow properties and improve stability of the composition by suspending materials commonly found in mascara, such as pigments. For example, clays that are reaction products of an organic quaternary amine with either hectorite or bentonite clay, are capable of swelling and gelling various hydrocarbon and natural oils, solvents and synthetic liquids in cosmetic compositions such as mascaras; see "Controlling Cosmetic Rheology," NL Industries (1985), p 6.

Japanese Patent Application 07-267,817 discloses waterproof oil-in-water mascara compositions comprising water soluble, water insoluble polymers, hydrophilic and hydrophobic clays in their respective internal and external phases of the compositions. However, it is known that mascaras containing clays in the external phase increase the viscosity in such a way that it becomes very difficult to wet the lashes with the mascara. This results in the consumer taking longer time to apply the mascara resulting in the lashes clumping.

EP-A-600445 discloses water-in-oil make-up cosmetic compositions comprising from 1 to 54% by weight of an oil-soluble resin in an external phase and 0.0001 to 63% by weight of a coloring material in an inner phase.

GB 2027341 discloses make-up foundation comprising an aqueous phase, said aqueous phase comprising from 0.03 to 1.0% by weight of a hydrophobic fumed silica and 0.03 to 0.5 % by weight of a hydrophilic fumed silica.

JP-A-63151351 discloses water-in-oil emulsions comprising water-swellable clay mineral, a non-ionic surfactant, an oil component and water as essential components.

DE-A-1949740 discloses a composition for the protection of the skin comprising a film-forming material (e.g. colloidal silica) and a gelling agent such as cholesterol.

"Magnesium Aluminium Silicate in Water-In-Oil Emulsion", DRUG & COSMETIC INDUSTRY discloses water-in-oil emulsions comprising magnesium aluminum silicate (MAS) in the internal phase (aqueous phase).

WO 98/18431, which represents prior art under Art. 54(3) EPC, discloses cosmetic oil-in-water compositions comprising organophilic clays.

### SUMMARY OF THE INVENTION

The present invention is for emulsion-form mascara compositions according to claim 1 providing surprising beauty and wear benefits as compared to compositions known in the art. These compositions comprise inorganic colloidal materials that are introduced into the internal phase of the emulsion composition. Said inorganic colloidal materials, naturally or treated to be compatible with the internal phase, are used in an amount necessary to provide good film strength for the applied composition. The addition of inorganic colloidal material in this manner, however, does not negatively impact the desired rheology of the composition.

All percentages are by weight of the cosmetic composition unless otherwise indicated. All solutions are on a weight/weight concentration unless otherwise indicated.

### DETAILED DESCRIPTION OF THE INVENTION

As used herein an emulsion composition means a composition comprising at least two distinct phases known as the internal phase and the external phase.

As used herein the term "internal phase" of the emulsion composition is the phase wherein the material or materials of said phase are dispersed as small particles within another distinct phase of the emulsion composition.

As used herein the term "external phase" of the emulsion composition is the phase wherein the internal phase is dispersed within.

### Inorganic Colloidal Material

The present invention relates to emulsion mascara compositions comprising from about 0.05% to 20% inorganic colloidal material in the internal phase of the emulsion. As previously disclosed inorganic colloidal material such as clays are known for use as compositional rheology modifiers. These materials, such as clays, are used to thicken or gel either or both the internal and, or the external phase of such compositions. However, it has been found that such inorganic colloidal material when incorporated into a composition in sufficient amounts can affect the film-forming character of the composition. Strengthening of formed films has been observed in the plastics industry; see Tie Lan and Dr. T.J. Pinnavaia (Dept. of Chemistry), CMS Courier, Vol I Issue 8, July 1994 ; "Polymer-Clay Nanocomposite Materials", pp.2-3 disclosing the addition of organophilic clays to nylon-6, improve the tensile strength, modulus, rheology and thermal capacity of plastics used in automobile production.

It has surprisingly been found that when inorganic colloidal materials, compatible with the internal phase of a mascara composition, are added to said internal phase, the resulting film formed upon the eyelashes has improved tensile strength, thereby improving the longevity of the composition. Furthermore, the rheology of the composition is not negatively impacted; i.e. the viscosity of the composition is not appreciably increased. Lastly, upon drying, the inorganic colloidal materials are complexed within the film rather than caking on the film. This avoids flaking of the composition from the lashes as the external phase evaporates.

The inorganic colloidal materials are used at levels from about 0.05% to about 20%, preferably 1% to about 10% and most preferably from about 2% to about 5% of the compositions of the present invention. The weight percentages above are based on the inorganic materials in neat form as opposed to the colloidal dispersion form that they may be purchased as. While these materials are commercially available, they are not necessarily limited to commercially available material.

The inorganic colloidal material can be incorporated into either oil or water provided such materials is either naturally compatible or can be modified or treated so as to be compatible with either oil or water. The inorganic colloidal material of the present invention is selected from the group consisting of amorphous silicon dioxide, oxides of aluminum and magnesium, hydroxides of aluminum and magnesium and mixtures thereof.

### 1. Amorphous Silicone Dioxide

Among the materials useful as the inorganic colloidal materials of the present invention are amorphous silicon dioxide. Amorphous silicon dioxide includes hydrated silica and silica.

### A. Hydrated Silica

Hydrated silica, a generic CTFA name applied to all synthetic silicon dioxides produced via a liquid process. The hydrated silica useful in the present invention includes precipitated silica and silica gel.
(1) Hydrophilic precipitated silica includes Zeothix 265, Zeosyl 200, Zeodent 163 and Zeofree 153 available from J. M. Huber.
(2) Hydrophobic precipitated silica is available from Tulco as Tullanox HM-250.
(3) Hydrophilic silica gel is formed in liquid medium, as a sheet, during manufacture and includes Sylox 2 and 15, Sylodent 2, 15, 700, and 704 available from W. R. Grace.

### B. Silica

Silica useful in the present invention is fumed silica. Fumed silica, also known as silica or pyrogenic silica is synthetic silicon dioxide typically prepared by steam hydrolysis of silicon tetrachloride at high temperatures.
(1) Hydrophobic pyrogenic silica products are obtained by reacting the silanol groups on the silica surface with chlorosilane. About 75% of the silanol groups on the surface are replaced by dimethyl silyl groups or modified with hexamethyldisilazane-surface treatment.
(2) Hydrophilic fumed silica is available from Cabot as Cab-o-sil M-5, H-5, HS-5, EH-5, L-90, and Cab-o-Sperse; Degussa as Aerosil 200, 200V, 300; Wacker-Chemie GmbH as Wacker HDK H20, N20, V15.
(3) Hydrophobic fumed silica is available from Degussa as Aerosil R812 and R972; and Cabot as Cab-o-sil TS-S30.

### 2. Oxides and Hydroxides of Aluminum and Magnesium

The oxides and hydroxides of aluminum and magnesium that are useful in the present invention include aluminum hydroxides, magnesium hydroxides, and aluminum/magnesium complex hydroxides.

### A. Aluminum Hydroxides

Aluminum hydroxides of the present invention are hydrated aluminum oxide pigments are white crystalline products of extremely fine and uniform particle size. The lattice of hydrated alumina consists of hydroxyl ions in coordination with an aluminum ion. Also, the amorphous hydrated aluminas which often coexist with the crystallized varieties in the preparation of alumina gels.
(1) Hydrophilic aluminum hydroxides of the present invention include Catapal⁺ Dispal Aluminas and Dispersal Alumina available from Vista Chemical Company.
(2) Hydrophobic aluminum hydroxides of the present are the same as those above, except the material is complexed with stearic acid.

### B. Aluminum/Magnesium Hydroxides

Aluminum/magnesium hydroxides are also useful in the present invention.
(1) Hydrophobic aluminum/magnesium hydroxides include Gilugel IPM, Gilugel IPP, Gilugel Min and Gilugel Sil 5, all available from Giulini.

### Additional Ingredients

Additional ingredients useful in the present invention are selected based on either the various forms or attributes the composition is to have. A list of such materials follows (said list is not all-encompassing):

### 1. Fatty Materials

The mascara compositions of the present invention can additionally include fatty materials. These materials may be in the internal phase, the external phase, or both the internal and external phases of the emulsion provided they are compatible with the phase they are introduced into. Preferably the solid fatty material are combined with other oily materials that go into the internal phase of a oil-in-water form of the present invention.

The fatty materials may comprises from about 0.05% to 50%, preferably from about 1.0% to 40%, and most preferably from about 2% to 25% by weight of the composition. The fatty materials are selected from the group consisting of waxes, fats, fatty acids, fatty alcohols and mixtures thereof.

### a. Waxes

Waxes are defined as lower-melting organic mixtures or compounds of high molecular weight, solid at room temperature and generally similar in composition to fats and oils except that they contain no glycerides. Some are hydrocarbons, others are esters of fatty acids and alcohols. Waxes useful in the present invention are selected from the group consisting of animal waxes, vegetable waxes, mineral waxes, various fractions of natural waxes, synthetic waxes petroleum waxes, ethylenic polymers, hydrocarbon types such as Fischer-Tropsch waxes, silicone waxes, and mixtures thereof wherein the waxes have a melting point between 40°C and 120°C and a needle penetration, as measured according to the American standard ASTM D5, of 3 to 40 at 25°C. The principle of the measurement of the needle penetration according to the standards ASTM D5 consists in measuring the depth, expressed in tenths of a millimeter, to which a standard needle (weighing 2.5 g and placed in a needle holder weighing 47.5 g, i.e. a total of 50 g) penetrates when placed on the wax for 5 seconds.

The specific waxes useful in the present invention are selected from the group consisting of beeswax, lanolin wax, shellac wax (animal waxes); carnauba, candelilla, bayberry (vegetable waxes); ozokerite, ceresin, (mineral waxes); paraffin, microcrystalline waxes (petroleum waxes); polyethylene, (ethylenic polymers); polyethylene homopolymers (Fischer-Tropsch waxes); C24-45 alkyl methicones (silicone waxes); synthetic waxes, and mixtures thereof. Most preferred are beeswax, lanolin wax, carnauba, candelilla, ozokerite, ceresin, paraffins, microcrystalline waxes, synthetic waxes, polyethylene, C24-45 alkyl methicones, and mixtures thereof.

### b. Fats

Fats useful in the present invention are triacylglyceride or triglyceride esters formed by an esterification reaction of fatty acids with glycerol. The fatty acids have carbon chain greater than about 12, such as stearic and palmitic fatty acids. The higher fatty acids used to form the fats are typically derived from marine, animals and plant sources. For more information regarding triglyceride oils, their sources and processing, refer to Bailey, "Industrial Oil and Fats Products". Interscience Publications; Such triglyceride esters are solids at room temperature and exhibit crystalline structure.

Fats are glyceryl esters of higher fatty acids such as stearic and palmitic. Such ester and their mixtures are solids at room temperature and exhibit crystalline structure.

The fats employed according to the invention are selected from the group consisting of fats derived from animals, vegetables, synthetically derived fats, and mixtures thereof wherein said fats have a melting point from about 40°C to about 100°C and a needle penetration, as measured according to the American standard ASTM D5, from about 3 to about 40 at 25°C. Preferably the fats selected for use in the present invention are selected from the group consisting of glyceryl monostearate, glyceryl distesrate, glyceryl tristearate, palmitate esters of glycerol, C18-36 triglycerides, glycerol monobehenate, glyceryl tribehenate and mixtures thereof.

### c. Fatty Acids and Alcohols

The fatty acids employed according to the invention are selected from the group consisting of fatty acids derived from animals, vegetables, synthetically derived fatty acids, and mixtures thereof. Preferably, the fatty acids selected for use in the present invention are selected from the group consisting of stearic acid, oleic acid, isostearic acid, palmitic acid, myristic acid, hydroxystearic acid, behenic acid, arachidic acid, lignoceric acid and lauric acid.

The fatty alcohols employed according to the invention are selected from the group consisting of fatty alcohols derived from animals, vegetables, synthetically derived fatty alcohols, and mixtures thereof wherein said fatty alcohols have a melting point from about 30°C to about 100°C and a needle penetration, as measured according to the American standard ASTM D5, from about 3 to about 40 at 25°C. Preferably, the fatty alcohols selected for the use in the present invention are selected from the group consisting of myristyl alcohol, cetyl alcohol, stearyl alcohol, hydroxystearyl alcohol, arachidyl alcohol, behenyl alcohol, and lignoceryl alcohol.

### 2. Film-Forming Polymers

The mascara composition that may be useful in the present invention may compsises various film forming polymeric materials to enhance the benefits of the present invention. Such film forming materials are disclosed in co-pending patent applications USSN 08/431,343, filed April 28, 1995; herein incorporated by reference. Such film-forming polymers include both water-soluble and water insoluble polymeric materials.

Water-insoluble materials useful in the present invention include latexes, or aqueous emulsions or dispersions of polymeric materials comprising polymers formed from monomers, said monomer derivatives, mixtures of said monomers, mixtures of said monomer derivatives, natural polymers and mixtures thereof. Said polymeric material also include chemically modified versions of the above polymers. These water-insoluble polymeric materials of the present invention comprise from about 3% to about 60% by weight of the composition. Other water-insoluble polymeric material include monomers selected from the group consisting of aromatic vinyls, dienes, vinyl cyanides, vinyl halides, vinylidene halides, vinyl esters, olefins and their isomers, vinyl pyrrolidone, unsaturated carboxylic acids, alkyl esters of unsaturated carboxylic acids, hydroxy derivatives of alkyl esters of unsaturated carboxylic acids, amides of unsaturated carboxylic acids, amine derivatives of unsaturated carboxylic acids, glycidyl derivatives of alkyl esters of unsaturated carboxylic acids, olefinic diamines and isomers, aromatic diamines, terephthaloyl halides, olefinic polyols and mixtures thereof. Specific polymeric material useful in the present invention include, but, are not necessarily limited to the Syntran Series (of latexes) from Interpolymer Corporation, for example Syntran 5170, Syntran EX33-1, Syntran EX30-1, and Syntran 5130 (acrylates copolymers formulated with added ammonia, propylene glycol, preservative and surfactant) and Syntran 5002 (styrene/acrylates/methacrylate copolymer formulated with added ammonia, propylene glycol, preservative and surfactant); the Primal Series (acrylic latexes) from Rohm & Haas; Appretan V (styrene/acrylic ester copolymer latexes) from Hoechst; Vinac (polyvinylacetate latex) from Air Products; UCAR latex resin 130 (polyvinylacetate latex) from Union Carbide; Rhodopas A Series (polyvinylacetate tatexes) from Rhone Poulenc; Appretan MB, EM, TV (vinyl acetate / ethylene copolymer latexes) from Hoechst; 200 Series (styrene/butadiene copolymer latexes) from Dow Chemical; Rhodopas SB Series (styrene/butadiene copolymer latexes) from Rhone Poulenc; Witcobond (polyurethane latexes) from Witco; Hycar Series (butadiene/acrylonitrile copolymer latexes) from Goodrich; Chemigum Series (butadiene/acrylonitrile copolymer latexes) from Goodyear; and Neo Cryl (styrene/acrylates/acrylonitrile copolymer latex) from ICI Resins.

Water-insoluble polymeric are those soluble in water, water-cosolvent mixtures, such as ethanol/water, pH adjusted water, and/or tempered solutions of the above to facilitate solubilization of the polymers. Water-soluble, film forming polymers comprise from about 0.1% to about 50%, preferably from about 1% to about 30%, and most preferably from about 1.5% to about 10% of the composition.

The film forming, water-soluble polymers comprise polymers formed from monomers, said monomer derivatives, mixtures of said monomers, mixtures of said monomer derivatives, natural polymers and mixtures thereof. The water-soluble, film forming polymers disclosed herein also include chemically modified versions of the above disclosed polymers. Said monomers are selected from the group consisting of olefin oxides, vinyl pyrrolidone, vinyl esters, vinyl alcohols, vinyl cyanides, oxazilines, carboxylic acids and esters and mixtures thereof. Preferred vinyl pyrrolidone polymers are selected from the group consisting of polyvinylpyrrolidone, vinyl acetate/vinyl pyrrolidone copolymer and mixtures thereof. Preferred polyvinyl esters are selected form the group consisting of vinyl acetate/crotonic acid copolymer, vinyl acetate/crotonic acid/vinyl neodecanoate copolymer and mixtures thereof. Preferred vinyl alcohol polymers are selected from the group consisting of vinyl alcohol/vinyl acetate, vinyl alcohol/poly(alkyleneoxy)acrylate, vinyl alcohol/vinyl acetate/poly(alkyleneoxy)acrylate and mixtures thereof. Preferred olefin oxides are selected from the group consisting of polyethylene oxide, polypropylene oxide and mixtures thereof. Preferred polycarboxylic acids and their esters are selected from the group consisting of acrylates, acrylatesloctylacrylamide copolymers and mixtures thereof. The preferred oxazilines is polyoxazilines.

Water-soluble, film forming polymers of the present invention comprise natural polymers selected from the group consisting of cellulose derivatives, algin and its derivatives, starch and its derivatives, guar and its derivatives, shellac polymers, and mixtures thereof. Preferred cellulose derivatives are selected from the group consisting of hydroxyethylcellulose, hydroxypropylcellulose, hydroxypropyl methylcellulose, ethylhydroxyethyl cellulose and mixtures thereof.

Specific water-soluble, film-forming polymers useful in the present invention include, but are not necessarily limited to Polyox WSR (polyethyleneoxide polymers) from Union Carbide; Natrosol 250 (hydroxyethylcellulose) from Aqualon; Cellosize (hydroxyethylcellulose) from Union Carbide; Airvol (polyvinylalcohol copolymer) from Air Products and Chemicals, preferably all commercially available grades like Airvol 103, Airvol 325, Airvol 540, Airvol 523S; Vinex from Air Products and Chemicals, preferably all commercially available grades such as Vinex 1003, Vinex 2034, Vinex 2144, Vinex 2019; PEOX (polyethyloxazoline) from Polymer Chemistry Innovations; PVP K Series (polyvinylpyrrolidone) from International Specialty Products; Luviskol K Series (polyvinylpyrrolidone) from BASF; PVP/VA (vinyl acetate/vinyl pyrrolidone copolymer) from International Specialty Products, preferably grades W-735 and S-630; and Gantrez (copolymers of methyl vinyl ether/maleic anhydride) from International Specialty Products; Carboset Series (acrylate copolymer) from BF Goodrich; Resyn Series (vinyl acetate/crotonate copolymers) from National Starch and Chemical Corporation; Versatyl and Dermacryl Series (acrylate/octylacrylamide copolymers) from National Starch and Chemical Corporation.

### 3. Emulsifiers

A component typically found in an emulsion composition is an emulsifier. In these embodiments of the present invention, emulsifiers are typically used at levels from about 0.1% to about 40%, preferably from about 0.5% to about 30%.

There are many factors which determine whether the water or the oil end up the dispersed or continuous phase. However, the single most important factor is the hydrophilic-lipophilic balance value (herein referred to as HLB) of the emulsifier; Wilkinson and Moore, Harry's Cosmeticology, 7th Ed. 1982, p. 738; Schick and Fowkes, Surfactant Science Series, Vol. 2, Solvent Properties of Surfactant Solutions, p 607. Said emulsifiers include those disclosed in the C.T.F.A. Cosmetic Ingrediem Handbook, 1992, pp. 587-592; and Remington's Pharmaceutical Sciences, 15th Ed. 1975, pp. 335-337; Said emulsifiers are selected from those known in the art and mixtures thereof including those in McCutcheon's Volume 1, Emulsifiers & Detergents, 1994, North American Edition, pp. 236-239

### 4. Pigments

The solids component of the mascara compositions of the present invention contain cosmetically acceptable pigments selected from the group consisting of inorganic pigments, organic pigments, and pearlescent pigments. When employed, the pigments are present in proportions depending on the color and the intensity of the color which it is intended to produce. The level of pigments in the solid portion of the mascara composition of present invention is from about 3% to about 30%, preferably from about 5% to about 20%. Pigments are selected from the group consisting of inorganic pigments, organic lake pigment., pearlescent pigments, and mixtures thereof. Said pigments may optionally be surface-treated within the scope of the present invention but are not limited to treatments such as silicones, perfluorinated compounds, lecithin, and amino acids.

Inorganic pigments useful in the present invention include those selected from the group consisting of rutile or anatase titanium dioxide, coded in the Color Index under the reference CI 77,891; black, yellow, red and brown iron oxides, coded under references CI 77,499, 77, 492 and, 77,491; manganese violet (CI 77,742); ultramarine blue (CI 77,007); chromium oxide (CI 77,288); chromium hydrate (CI 77,289); and ferric blue (CI 77,510) and mixtures thereof.

The organic pigments and lakes useful in the present invention include those selected from the group consisting of D&C Red No. 19 (CI 45,170), D&C Red No. 9 (CI 15,585),D&C Red NO. 21 (CI 45,380), D&C Orange No. 4 (CI 15,510), D&C Orange No. 5 (CI 45,370), D&C Red No. 27 (CI 45,410), D&C Red No. 13 (CI 15,630), D&C Red No. 7 (CI 15,850), D&C Red No. 6 (CI 15,850), D&C Yellow No. 5 (CI 19,140), D&C Red No. 36 (CI 12,085), D&C Orange No. 10 (CI 45,425), D&C Yellow No. 6 (CI 15,985), D&C Red No. 30 (CI 73,360), D&C Red No. 3 (CI 45,430) and the dye or lakes based on Cochineal Carmine (CI 75,570) and mixtures thereof.

The pearlescent pigments useful in the present invention include those selected from the group consisting of the white pearlescent pigments such as mica coated with titanium oxide, bismuth oxychloride, colored pearleseent pigments such as titanium mica with iron oxides, titanium mica with ferric blue, chromium oxide and the like, titanium mica with an organic pigment of the above-mentioned type as well as those based on bismuth oxychloride and mixtures thereof.

### 5. Volatile Fluids

Volatile fluids are particularly useful optional ingredient. Said volatile fluids are selected from the group consisting of volatile hydrocarbons, volatile silicones and mixtures thereof. Volatile hydrocarbon fluids that may be used in the present invention include isododecane, peholeum distillates, and isoparaffins. Preferred are isododocane and petroleum distillates. Isododecane is available as for example Permethyl 99A from Permethyl Corporation corresponding to the formula:

CH₃(CH₂)₁₀CH₃

Volatile silicone fluids include cyclomethicones having 3, 4 and 5 membeced ring structutes corresponding to the formula: 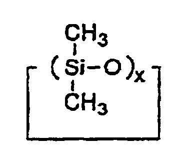 where X is from about 3 to about 6. Said volatile silicones include 244 Fluid, 344 Fluid and 345 Fluid from Dow Corning Corporation.

In an oil-in-water mascara emulsion composition, volatile fluids, such as the volatile silicone, and fatty materials are gelled with a hydrophobically-modified fumed silica powder in the internal phase of the composition. In another embodiment of the present invention, a volatile fluid may be used within the external phase of a water-in-oil composition to extend the fatty materials so that a large volume external phase is created for emulsifying the water/inorganic colloid slurry.

### 6. Miscellaneous

In the present invention numerous optional ingredients may be added to provide additional benefits other than that attributed to the invention as defined above. For example, it is preferred that the mascara composition of the present invention contain a preservative system to inhibit microbiological growth and maintain the integrity of the product. In the present invention, the preservative system does not have a detrimental effect on the composition.

Any optional ingredients known to those skilled in the art may also be used in the invention. Examples of optional ingredients are cosmetic fillers including, but not limited to, mica, talc, nylon, polyethylene, silica beads, polymethacrylate, kaolin, teflon; cosmetic preservatives including, but not limited to, methylparaben, propylparaben, butylparaben, ethylparaben, potassium sorbate, trisodium EDTA, phenoxyethanol, ethyl alcohol, diazolidinyl urea, benzyl alcohol, imidazolidinyl urea, quaternium-15. Also, additives such as tall oil glycerides are easily incorporated into emulsion forms of the mascara.

| Example #1 | |
|---|---|
| **Ingredient** | **W/W%** |
| Deionized Water | 62.69 |
| Paraffin Wax | 4.00 |
| Glycerol Behenate | 2.00 |
| Beeswax | 2.00 |
| Carnauba Wax | 4.50 |
| Black Iron Oxide | 9.25 |
| Fumed Silica¹ | 0.75 |
| Quaternium-18 Hectorite² | 2.00 |
| Propylene Carbonate | 1.50 |
| Stearic Acid | 2.75 |
| Oleic Acid | 0.75 |
| Triethanolamine | 1.75 |
| Trisodium EDTA | 0.10 |
| Propylene Glycol | 2.00 |
| Lecithin | 1.25 |
| Ethyl Alcohol³ | 1.00 |
| Benzyl Alcohol | 0.65 |
| Phenoxyethanol | 0.28 |
| Propylparaben | 0.10 |
| Methylparaben | 0.20 |
| Ethylparaben | 0.20 |
| Panthenol⁴ | 0.28 |

| | |
|---|---|
| 1. available as Aerosil R 972 from Degussa | |
| 2. available as Bentone 38 from Rheox. | |
| 3. available as SD Alcohol 40-B from Warner Graham Company. | |
| 4. available as dl-Panthenol from Roche. | |

### PROCESSING DIRECTIONS

Place the waxes and fats into a vessel equipped with heating and mixing. Heat the waxes and fats with low speed mixing to a temperature sufficient to liquefied the mixture. Continue mixing until homogeneous. Add oil-dispersible or oil-soluble components such as pigments. Increase the mixing rate to high and mix until the pigments are uniformly dispersed throughout the lipid mixture; about 30-35 minutes. Add emulsifiers to said lipid mixture while continuing to mix .

In a second vessel equipped with mixing and heating, add water followed by any water-soluble, film-forming polymers used, and the remainder of the water-dispersible components. The mixture of water and water-soluble film forming polymers can be made up ahead of the processing of the mascara composition. Mix with heating until this aqueous mixture is about 90- 95°C. Q.S. for any water loss from said aqueous mixture.

Slowly combine the two mixtures and mix with a high speed dispersator type mixer. Remove heat source and continue mixing this combined mixture until the temperature of said combined mixture is from about 65°C-70°C. Q.S. said combined mixture for any water loss, add the preservatives and insoluble polymer component and mix until homogeneous. Cool said combined mixture to about 45-47°C. Add any remaining components. Continue cooling and mixing until said combined mixture is about 27- 30°C. Transfer said combined mixture to suitable storage containers for subsequent filling of retail size packaging.

## Claims

1. An oil-in-water emulsion mascara composition comprising in the internal phase from 0.05% to 20.0%, preferably 1% to .10%, and most preferably from 2% to 5% by weight of the composition of a hydrophobic inorganic colloidal material selected from the group consisting of amorphous silicon dioxide, oxides of aluminum and magnesium, hydroxides of aluminum and magnesium and mixtures thereof.

2. A mascara composition according to claim 1, additionally comprising from 0.05% to 50%, preferably 1.0% to 40% fatty material selected from the group consisting of waxes, fats, fatty acids, fatty alcohols, and mixtures thereof preferably waxes selected from the group consisting of animal waxes, vegetable waxes, mineral waxes, various fractions of natural waxes, synthetic waxes petroleum waxes, ethylenic polymers, hydrocarbon types such as Fischer-Tropsch waxes, silicone waxes, and mixtures thereof wherein the waxes have a melting point between 40°C and 120°C and a needle penetration, as measured according to the American standard ASTM D5, of 3 to 40 at 25°C, most preferably selected from the group consisting of beeswax, lanolin wax, carnauba, candelilla, ozokerite, ceresin, paraffins, microcrystalline waxes, synthetic waxes, polyethylene, C24-45 alkyl methicones and mixtures thereof.

## Patentansprüche

1. Mascarazusammensetzung in Form einer Öl-in-Wasser-Emulsion, umfassend in der inneren Phase 0,05% bis 20,0%, vorzugsweise 1% bis 10%, und am meisten bevorzugt 2% bis 5%, bezogen auf Gewicht der Zusammensetzung, eines hydrophoben, anorganischen, kolloidalen Materials, gewählt aus der Gruppe, bestehend aus amorphem Siliciumdioxid, Oxiden von Aluminium und Magnesium, Hydroxiden von Aluminium und Magnesium und Mischungen hiervon.

2. Mascarazusammensetzung nach Anspruch 1, umfassend zusätzlich 0,05% bis 50%, vorzugsweise 1,0% bis 40% Fettmaterial, gewählt aus der Gruppe, bestehend aus Wachsen, Fetten, Fettsäuren, Fettalkoholen und Mischungen hiervon, vorzugsweise Wachsen, gewählt aus der Gruppe, bestehend aus tierischen Wachsen, pflanzlichen Wachsen, Mineralwachsen, verschiedenen Fraktionen von natürlichen Wachsen, synthetischen Wachsen, Erdölwachsen, ethylenischen Polymeren, Kohlenwasserstofftypen, wie Fischer-Tropsch-Wachsen, Siliconwachsen und Mischungen hiervon, wobei die Wachse einen Schmelzpunkt zwischen 40°C und 120°C und eine Nadelpenetration, gemessen gemäß dem amerikanischen Standard ASTM D5, von 3 bis 40 bei 25°C aufweisen, am meisten bevorzugt gewählt aus der Gruppe, bestehend aus Bienenwachs, Lanolinwachs, Karnauba, Kandelilla, Ozokerit, Ceresin, Paraffinen, mikrokristallinen Wachsen, synthetischen Wachsen, Polyethylen, C₂₄₋₄₅-Alkylmethiconen und Mischungen hiervon.

## Revendications

1. Composition de mascara de type émulsion huile dans l'eau comprenant dans la phase interne 0,05 % à 20,0 %, de préférence 1 % à 10 %, et au mieux 2 % à 5% en poids de la composition d'une matière colloïdale inorganique hydrophobe choisie dans le groupe constitué par le dioxyde de silicium amorphe, les oxydes d'aluminium et de magnésium, les hydroxydes d'aluminium et de magnésium, et leurs mélanges.

2. Composition de mascara selon la revendication 1 comprenant en outre 0,05 à 50 % de préférence 1,0 à 40 % d'une matière grasse choisie dans le groupe constitué par les cires, les graisses, les acides gras, les alcools gras et leurs mélanges, de préférence les cires choisies dans le groupe constitué par les cires d'origine animale, les cires d'origine végétale, les cires d'origine minérale, les diverses fractions de cires naturelles, les cires d'origine synthétique, les cires pétrole, les polymères d'éthylène, les cires de type hydrocarbures telles que les cires Fischer-Tropsch, les cires silicones, et leurs mélanges, où les cires présentent un point de fusion compris entre 40 °C et 120 °C et une pénétration d'aiguille, telle que mesurée en accord avec la norme américaine ASTDM D5, comprise entre 3 et 40 à 25 °C, idéalement choisie dans le groupe constitué par la cire d'abeilles, la cire de lanoline, la cire de carnauba, la cire de candellila, l'ozokérite, la cérésine, les paraffines, les cires microcristallines, les cires d'origine synthétique, le polyéthylène, les alkyle en C₂₄ à C₄₅-méthicones et leurs mélanges.
